# EUROPÄISCHE PATENTSCHRIFT

(11) **EP 3 466 482 B1**
(45) Veröffentlichungstag und Bekanntmachung des Hinweises auf die Patenterteilung: **29.01.2020**
(21) Anmeldenummer: 18190374.1
(22) Anmeldetag: 23.08.2018
(51) Int. Cl.: A61M 39/24

(54) **VENTILVORRICHTUNG FÜR EIN MEDIZINISCHES FLUIDLEITSYSTEM**
VALVE DEVICE FOR A MEDICAL FLUID GUIDANCE SYSTEM
DISPOSITIF DE SOUPAPE POUR UN SYSTÈME DE CONDUITES DE FLUIDE MÉDICAL

(30) Priorität: 04.10.2017 DE 102017217634
(43) Veröffentlichungstag der Anmeldung: 10.04.2019
(73) Patentinhaber: B. Braun Melsungen AG, 34212 Melsungen (DE)
(72) Erfinder: Kunschak, Ralf, 6130 Willisau (CH)
(74) Vertreter: Patentanwälte Ruff, Wilhelm, Beier, Dauster & Partner mbB

(56) Entgegenhaltungen:
- EP-B1- 1 954 343
- WO-A1-92/06732
- US-A- 2 986 098
- US-A- 3 822 720
- US-A- 4 332 249
- US-A- 5 224 938
- US-A1- 2013 160 866

## Beschreibung

Die Erfindung betrifft eine Ventilvorrichtung mit den Merkmalen des Oberbegriffs von Anspruch 1.

Eine derartige Ventilvorrichtung ist aus der US 2013/0160866 A1 bekannt und für einen Venenkatheter vorgesehen. Solche Katheter werden üblicherweise zur intravenösen Verabreichung oder Entnahme einer medizinischen Flüssigkeit bzw. einer Körperflüssigkeit verwendet. Dabei wird die entsprechende Flüssigkeit überdruckbedingt aus dem Katheter injiziert oder unterdruckbedingt in diesen aspiriert. Bei einem Nichtgebrauch des Katheters ist es nicht ausgeschlossen, dass Körperflüssigkeit ungewollt in den Katheter eindringt. Wird dieser nicht ausgespült, kann der Katheter verstopfen. Die bekannte Ventilvorrichtung versucht dem entgegenzuwirken und sieht hierzu - je nach Über- oder Unterdruckbeaufschlagung des Katheters - wechselseitig abdicht- und freigebbare Fluiddurchlässe vor. Diese sind jeweils schlitzförmig in einen Grundkörper in Form eines Silikonformteils eingebracht. Bei einem injektionsbedingten Überdruck ist ein erster der Schlitze freigegeben, wohingegen ein zweiter der Schlitze abgedichtet ist. Bei einem aspirationsbedingten Unterdruck ist der zweite Schlitz freigegeben, wohingegen der erste Schlitz abgedichtet ist. Bei Nichtgebrauch des Katheters sind die Fluiddurchlässe abgedichtet. Das Freigeben und Abdichten der Schlitze wird mittels einer fluiddruckbedingten Deformation des Silikonformteils bewirkt. Bei der bekannten Ventilvorrichtung sind die Schlitze zueinander benachbart in einem trichterförmigen Abschnitt des Silikonformteils angeordnet.

Eine weitere derartige Ventilvorrichtung ist aus der WO 92/06732 A1 bekannt und an einem medizinischen Katheter vorgesehen. Die bekannte Ventilvorrichtung weist einen Grundkörper sowie einen ersten Fluiddurchlass und einen zweiten Fluiddurchlass auf. Die Fluiddurchlässe sind zwischen einer Einlass- und einer Auslassseite des Grundkörpers erstreckt. Der Grundkörper ist durch eine Art Stegabschnitt gebildet. Seitlich des Stegabschnitts ragen zu beiden Seiten Wandabschnitte ab. Die Wandabschnitte liegen stirnendseitig an einer Innenseite eines Fluidleitungsabschnitts des Katheters an und bilden auf diese Weise jeweils einen der Fluiddurchlässe aus. Die Fluiddurchlässe sind je nach Strömungsrichtung bzw. Druckbeaufschlagung wechselweise freigegeben oder abgedichtet.

Aufgabe der Erfindung ist es, eine Ventilvorrichtung der eingangs genannten Art zu schaffen, die eine verbesserte Dichtfunktion und einen einfachen Aufbau ermöglicht.

Diese Aufgabe wird durch eine Ventilvorrichtung mit den Merkmalen des Anspruchs 1 gelöst. Durch die erfindungsgemäße Lösung ist es insbesondere möglich, die Eigenschaften der Ventilvorrichtung bei einer Überdruckbeaufschlagung im Wesentlichen unabhängig von den Eigenschaften bei einer Unterdruckbeaufschlagung zu gestalten. Denn jedem der Fluiddurchlässe ist ein gesonderter Wandabschnitt zugeordnet. Dies ermöglicht - vereinfacht ausgedrückt - eine verbesserte konstruktive Auslegbarkeit der wechselseitigen Dichtfunktion. So kann beispielsweise der dem ersten Fluiddurchlass zugeordnete Wandabschnitt elastisch nachgiebiger verlagerbar sein als der dem zweiten Fluiddurchlass zugeordnete Wandabschnitt oder umgekehrt. Auf diese Weise kann beispielsweise der Überdruck, bei welchem der erste Wandabschnitt den ersten Fluiddurchlass freigibt, konstruktionsseitig unabhängig bemessen werden von dem Unterdruck, bei welchem der zweite Wandabschnitt den zweiten Fluiddurchlass freigibt und umgekehrt. Zudem ermöglicht die erfindungsgemäße Lösung eine verbesserte konstruktionsseitige Anpassbarkeit der Volumenströme in Abhängigkeit der Durchströmungsrichtung der Ventilvorrichtung. Der Stegabschnitt ist vorzugsweise parallel zu einer Hauptdurchströmungsrichtung der Ventilvorrichtung längserstreckt. Die Wandabschnitte sind relativ zu dem Stegabschnitt jeweils zwischen einer Freigabeposition, in der der jeweilige Wandabschnitt den jeweiligen Fluiddurchlass freigibt, und einer Abdichtposition, in der der jeweilige Wandabschnitt den jeweiligen Fluiddurchlass abdichtet, fluiddruckbedingt elastisch verlagerbar. Die Wandabschnitte können beispielsweise insoweit elastisch verlagerbar gestaltet sein, als dass sie jeweils wenigstens abschnittsweise aus einem gummielastischen Werkstoff gefertigt sind oder im Wesentlichen aus einem unelastischen Werkstoff gefertigt und dabei elastisch nachgiebig an dem Grundkörper abgestützt sind. Dass der erste Fluiddurchlass im Wesentlichen unbeeinflusst von einer fluiddruckbedingten Deformation des zweiten Wandabschnitts ist, meint, dass eine bestehende Abdichtung oder Freigabe des ersten Fluiddurchlasses mittels einer bestimmungsgemäßen Deformation des zweiten Wandabschnitts im Wesentlichen nicht beeinflussbar ist. Entsprechendes gilt für den zweiten Fluiddurchlass im Zusammenhang mit dem ersten Wandabschnitt.

Die erfindungsgemäße Lösung eignet sich in besonders bevorzugter Weise für eine Ventilvorrichtung zur Anordnung in einem Katheter, insbesondere in einem peripher eingeführten zentralvenösen Katheter. Die erfindungsgemäße Lösung eignet sich jedoch auch zur Anordnung in einem Lumen einer beliebigen medizinischen Fluidleitungskomponente, wie sie üblicherweise bei einer Infusionstherapie verwendet wird.

Weiter gemäß der Erfindung sind die Wandabschnitte an einander gegenüberliegenden Seitenflächen des Stegabschnitts und somit getrennt voneinander angeordnet. Die Seitenflächen sind vorzugsweise parallel zu der Hauptdurchströmungsrichtung der Ventilvorrichtung ausgerichtet. Mittels der auf diese Weise voneinander getrennten Anordnung der Wandabschnitte wird insbesondere erreicht, dass bei einer fluiddruckbedingten Deformation eines der Wandabschnitte lediglich der diesem Wandabschnitt zugeordnete Fluiddurchlass abgedichtet und/oder freigegeben wird.

In weiterer Ausgestaltung der Erfindung liegen die Wandabschnitte - in Bezug auf einen abgedichteten Zustand - jeweils wenigstens abschnittsweise nach Art einer Dichtlippe an dem Stegabschnitt an, wobei jeweils einer der Fluiddurchlässe in Form eines Schlitzes zwischen dem Stegabschnitt, insbesondere der jeweiligen Seitenfläche, und dem jeweiligen Wandabschnitt ausgebildet ist. Demgegenüber sind die Wandabschnitte - in Bezug auf einen freigegebenen Zustand - jeweils in Normalenrichtung des Stegabschnitts relativ zu diesem elastisch verlagert. Sofern die Wandabschnitte an einander gegenüberliegenden Seitenflächen des Stegabschnitts angeordnet sind, liegen die Wandabschnitte vorzugsweise an der jeweiligen Seitenfläche an (Abdichtungsposition) bzw. sind von dieser abgehoben (Freigabeposition).

In weiterer Ausgestaltung der Erfindung ist der erste Fluiddurchlass zwischen einem der Auslassseite zugewandten Stirnende des ersten Wandabschnitts und dem Stegabschnitt ausgebildet und der zweite Fluiddurchlass ist zwischen einem der Einlassseite zugewandten Stirnende des zweiten Wandabschnitts und dem Stegabschnitt ausgebildet. Demzufolge sind die Fluiddurchlässe vorzugsweise an einander gegenüberliegenden Stirnenden des Stegabschnitts angeordnet.

In weiterer Ausgestaltung der Erfindung überspannen die Wandabschnitte den Stegabschnitt, insbesondere die jeweilige Seitenfläche, wenigstens abschnittsweise gewölbt unter Ausbildung jeweils eines Lumens, wobei die Lumen jeweils einends in den jeweiligen Fluiddurchlass münden. Demnach sind die Wandabschnitte jeweils flächig gegenüber dem Stegabschnitt, insbesondere der jeweiligen Seitenfläche, angeordnet und an wenigstens zwei gegenüberliegenden Seiten fluiddicht mit dem Stegabschnitt, insbesondere der jeweiligen Seitenfläche, verbunden. Auf diese Weise wird jeweils eines der Lumen ausgebildet. Die Lumen erstrecken sich jeweils zwischen der Einlass- und der Auslassseite des Grundkörpers.

In weiterer Ausgestaltung der Erfindung sind die Lumen jeweils nach Art einer Strömungsdüse geformt und derart strömungstechnisch entgegengesetzt zueinander orientiert, dass eine Einlassöffnung des dem ersten Wandabschnitt zugeordneten Lumens in Richtung der Einlassseite orientiert ist und eine Einlassöffnung des dem zweiten Wandabschnitt zugeordneten Lumens in Richtung der Auslassseite orientiert ist, wobei die Fluiddurchlässe jeweils eine Auslassöffnung des jeweiligen Lumens bilden. Nach Art einer Strömungsdüse geformt meint insbesondere, dass die Lumen jeweils einen - in Bezug auf eine Hauptdurchströmungsrichtung der Ventilvorrichtung - veränderlichen Strömungsquerschnitt aufweisen können. Vorzugsweise sind die Strömungsquerschnitte ausgehend von der jeweiligen Einlassöffnung jeweils in Richtung der jeweiligen Auslassöffnung bzw. des jeweiligen Fluiddurchlasses verjüngt.

In weiterer Ausgestaltung der Erfindung ist der Stegabschnitt - im Vergleich zu den Wandabschnitten - steif gestaltet und im Wesentlichen entlang einer Mittellängsachse des Grundkörpers in Form einer ebenen Platte erstreckt. Die ebene Platte weist vorzugsweise eine rechteckige Grundform auf, wobei die Wandabschnitte jeweils an einer Vorder- bzw. einer Rückseite der Platte getrennt voneinander angeordnet sein können.

In weiterer Ausgestaltung der Erfindung sind die Wandabschnitte derart unterschiedlich elastisch verlagerbar gestaltet, dass die Fluiddurchlässe bei betragsmäßig unterschiedlichen Fluiddrücken freigegeben und/oder abgedichtet sind. Je nach praktischem Anwendungsfall kann sich die Viskosität einer durch den ersten Fluiddurchlass zu injizierenden Flüssigkeit von der Viskosität einer durch den zweiten Fluiddurchlass zu aspirierenden Flüssigkeit unterscheiden. Damit einhergehend kann es erforderlich sein, die Fluiddurchlässe derart unterschiedlich zu gestalten, dass diese bei, insbesondere deutlich, unterschiedlichen Fluiddrücken freigegeben und/oder abgedichtet werden. Diese Ausgestaltung der Erfindung ermöglicht eine einfache dahingehende Anpassbarkeit der Ventilvorrichtung.

In weiterer Ausgestaltung der Erfindung weisen die Wandabschnitte unterschiedliche Wandstärken, Wandstärkenverläufe und/oder elastische Eigenschaften auf. Beispielsweise kann der erste Wandabschnitt im Vergleich zu dem zweiten Wandabschnitt hinsichtlich seiner Wandstärke dünnwandiger gestaltet sein oder umgekehrt. Alternativ oder zusätzlich ist es möglich, dass die Wandabschnitte - vorzugsweise in Bezug auf die Hauptdurchströmungsrichtung der Ventilvorrichtung - unterschiedliche Wandstärkenverläufe aufweisen. Alternativ oder zusätzlich ist es auch möglich, dass der erste Wandabschnitt aus einem anderen Werkstoff gefertigt ist als der zweite Wandabschnitt, so dass diese unterschiedliche elastische Eigenschaften aufweisen. Diese Ausgestaltung der Erfindung ermöglicht eine einfache Anpassbarkeit des zum Verlagern des jeweiligen Wandabschnitts zwischen der Abdicht- und der Freigabeposition notwendigen Fluiddrucks.

In weiterer Ausgestaltung der Erfindung sind die Wandabschnitte betragsmäßig unterschiedlich gegenüber dem Stegabschnitt geneigt und/oder unterschiedlich längserstreckt, so dass eine - senkrecht zu der Einlassseite - projizierte Druckfläche des ersten Wandabschnitts unterschiedlich zu einer - senkrecht zu der Auslassseite - projizierten Druckfläche des zweiten Wandabschnitts ist. Die jeweilige Neigung und/oder Längserstreckung beeinflusst demnach die projizierte Druckfläche und somit den Fluiddruck, bei welchem der jeweilige Wandabschnitt fluiddruckbedingt elastisch verlagert wird. Diese Ausgestaltung der Erfindung ermöglicht eine besonders bedarfsgerechte konstruktionsseitige Anpassbarkeit der Ventilvorrichtung.

In weiterer Ausgestaltung der Erfindung weist der Grundkörper einen ringförmigen Profilabschnitt auf, der zur formschlüssigen Verbindung mit einem Innendurchmesser einer medizinischen Fluidleitungskomponente vorgesehen ist. Der Profilabschnitt kann in Form einer ringförmigen Nut oder in Form einer ringförmigen Auswölbung an dem Grundkörper angeordnet sein. Vorzugsweise ist der Profilabschnitt radial zu einer Mittellängsachse des Grundkörpers orientiert.

In weiterer Ausgestaltung der Erfindung umgreift der Profilabschnitt den Stegabschnitt - in Bezug auf eine Längserstreckung des Stegabschnitts - in etwa mittig wenigstens abschnittsweise in Umfangsrichtung. Demzufolge ist der Stegabschnitt vorzugsweise durch eine gedachte Durchgangsöffnung des ringförmigen Profilabschnitts erstreckt.

In weiterer Ausgestaltung der Erfindung sind die Wandabschnitte jeweils an einem dem jeweiligen Fluiddurchlass abgewandten Stirnende mit dem Profilabschnitt verbunden. Demnach ist der erste Wandabschnitt an seinem der Einlassseite zugewandten Stirnende mit dem Profilabschnitt verbunden. Demgegenüber ist der zweite Wandabschnitt an seinem der Auslassseite zugewandten Stirnende mit dem Profilabschnitt verbunden. Diese Ausgestaltung der Erfindung ist insbesondere besonders fertigungsgerecht.

In weiterer Ausgestaltung der Erfindung ist der Grundkörper einstückig ausgebildet. Beispielsweise können die Wandabschnitte zusammen mit dem Stegabschnitt in Form eines einstückigen Kunststoffspritzgussbauteils gefertigt sein. Soweit zusätzlich ein Profilabschnitt vorgesehen ist, ist dieser vorzugsweise einstückig mit den verbleibenden Komponenten des Grundkörpers, insbesondere den Wandabschnitten und dem Stegabschnitt, verbunden. Der Grundkörper kann beispielsweise einstückig aus ein- und demselben gummielastischen Werkstoff gefertigt sein. Alternativ ist es möglich, dass der Stegabschnitt und die Wandabschnitte aus unterschiedlichen Werkstoffen gefertigt und hiernach einstückig stoffschlüssig miteinander verbunden sind.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in einer schematischen Perspektivdarstellung eine Ausführungsform einer erfindungsgemäßen Ventilvorrichtung,
- Fig. 2: in einer schematischen Längsschnittdarstellung die Ventilvorrichtung nach Fig. 1 in einem in einem Lumen einer Fluidleitungskomponente angeordneten Einbauzustand,
- Fig. 3: in einer schematischen Längsschnittdarstellung ähnlich zu Fig. 2 eine weitere Ausführungsform einer erfindungsgemäßen Ventilvorrichtung und
- Fig. 4: in einer Längsschnittdarstellung eine weitere Fluidleitungskomponente eines medizinischen Fluidleitungssystems mit einer ersten und einer zweiten Ventilvorrichtung nach den Fig. 1 bis 3.

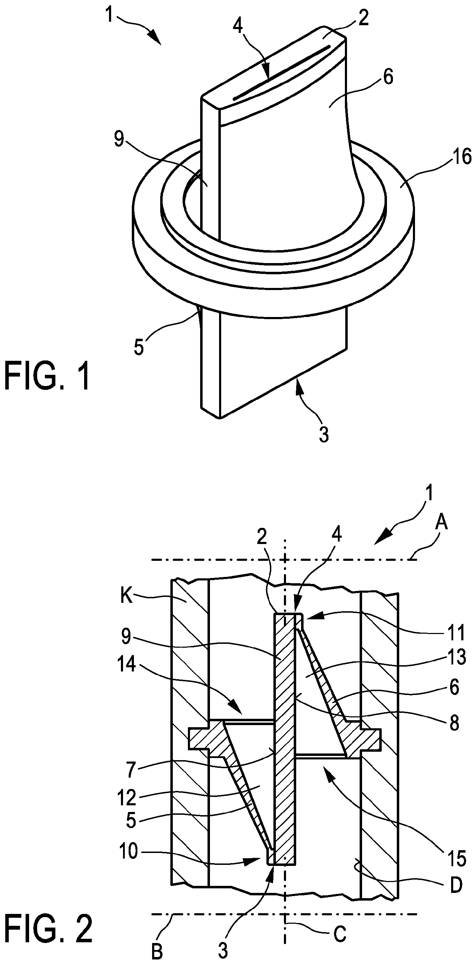

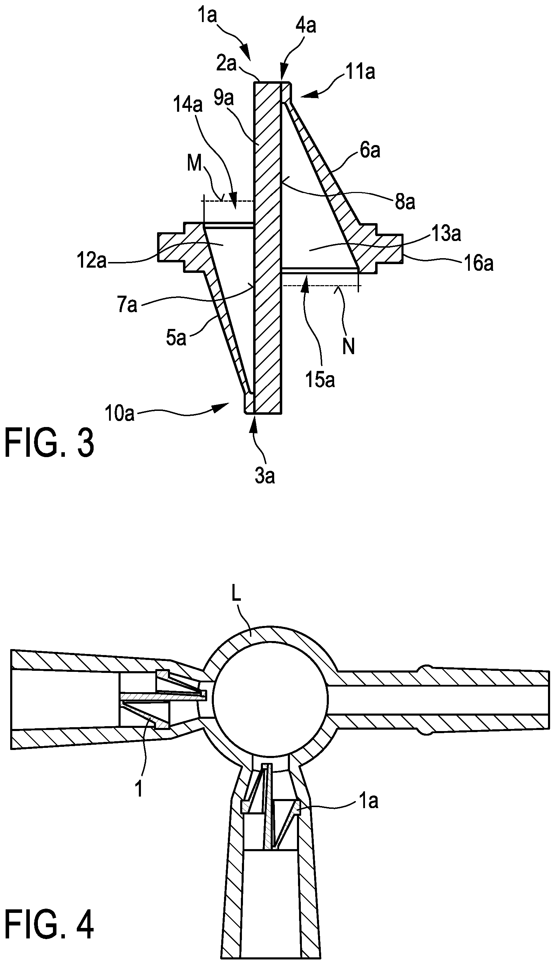

Die Ventilvorrichtungen 1, 1a nach den Fig. 1, 2 und 4 bzw. Fig. 3 und 4 sind zur Steuerung eines Fluidflusses durch ein Lumen einer Fluidleitungskomponente K, L (vgl. Fig. 2 und 4) eines Fluidleitungssystems vorgesehen.

Gemäß Fig. 1 weist die Ventilvorrichtung 1 einen Grundkörper 2 auf, der wenigstens abschnittsweise weichelastisch gestaltet ist. Vorliegend ist der Grundkörper 2 einstückig aus einem gummielastischen Werkstoff, beispielsweise einem Silikonelastomer gefertigt. Weiter sieht die Ventilvorrichtung 1 einen ersten Fluiddurchlass 3 sowie einen zweiten Fluiddurchlass 4 vor, die jeweils zwischen einer Einlassseite A und einer Auslassseite B des Grundkörpers 2 erstreckt sind. Die Einlassseite A und die Auslassseite B sind anhand Fig. 2 jeweils in Form strichlierter Linien schematisch ersichtlich. Der Grundkörper 2 weist einen ersten Wandabschnitt 5 und einen zweiten Wandabschnitt 6 auf, die an einander gegenüberliegenden Seitenflächen 7, 8 eines Stegabschnitts 9 des Grundkörpers 2 angeordnet sind. Die Wandabschnitte 5, 6 sind auf noch näher zu beschreibende Art und Weise jeweils relativ zu dem Stegabschnitt 9 bzw. der jeweiligen Seitenfläche 7 oder 8 fluiddruckbedingt elastisch verlagerbar. Demgegenüber ist der Stegabschnitt 9 im Vergleich zu den Wandabschnitten 5, 6 formstabil gestaltet und im Wesentlichen entlang einer Mittellängsachse C des Grundkörpers 2 erstreckt. Wie insbesondere anhand Fig. 1 ersichtlich ist, ist der Stegabschnitt 9 in Form einer ebenen Platte ausgebildet. Vorliegend ist die Platte 9 rechteckig geformt, was jedoch nicht notwendigerweise der Fall sein muss.

Der erste Fluiddurchlass 3 ist zwischen einem der Auslassseite B zugewandten Stirnende 10 des ersten Wandabschnitts 5 und dem Stegabschnitt 9, genauer: der Seitenfläche 7, ausgebildet. Demgegenüber ist der zweite Fluiddurchlass 4 zwischen einem der Einlassseite A zugewandten Stirnende 11 des zweiten Wandabschnitts 6 und dem Stegabschnitt 9, genauer: der Seitenfläche 8, ausgebildet. Dabei liegen die Wandabschnitte 5, 6 mit ihrem jeweiligen Stirnende 10, 11 jeweils wenigstens abschnittsweise nach Art einer Dichtlippe an dem Stegabschnitt 9 an. Auf diese Weise sind die Fluiddurchlässe 3, 4 jeweils in Form eines Schlitzes zwischen dem Stegabschnitt 9 und dem jeweiligen Wandabschnitt 5, 6 ausgebildet. Wie weiter anhand Fig. 2 ersichtlich ist, überspannen die Wandabschnitte 5, 6 die jeweilige Seitenfläche 7, 8 des Stegabschnitts 9 wenigstens abschnittsweise gewölbt (vgl. Fig. 1). Auf diese Weise ist jeweils ein Lumen 12, 13 ausgebildet. Das zwischen dem ersten Wandabschnitt 5 und der Seitenfläche 7 ausgebildete Lumen 12 mündet einends in den ersten Fluiddurchlass 3. Das zwischen dem zweiten Wandabschnitt 6 und der Seitenfläche 8 ausgebildete Lumen 13 mündet einends in den zweiten Fluiddurchlass 4. Die Lumen 12, 13 sind jeweils nach Art einer Strömungsdüse geformt und weisen insoweit jeweils einen sich in Richtung des jeweiligen Fluiddurchlasses 3, 4 verjüngenden Strömungsquerschnitt auf (vgl. Fig. 2). Dabei sind die Lumen 12, 13 dahingehend strömungstechnisch entgegengesetzt zueinander orientiert, als dass eine Einlassöffnung 14 des ersten Lumens 12 in Richtung der Einlassseite A orientiert ist und eine Einlassöffnung 15 des Lumens 13 demgegenüber in Richtung der Auslassseite B orientiert ist.

Weiter weist der Grundkörper 2 einen ringförmigen Profilabschnitt 16 auf. Der ringförmige Profilabschnitt 16 ist insbesondere zur formschlüssigen Verbindung mit einem Innendurchmesser D einer medizinischen Fluidleitungskomponente K vorgesehen, wie anhand Fig. 2 ersichtlich ist. Der Profilabschnitt 16 umgreift den Stegabschnitt 9 - in Bezug auf eine Längserstreckung des Stegabschnitts 9 - in etwa mittig und in Umfangsrichtung vollständig. Die Wandabschnitte 5, 6 sind jeweils an einem dem jeweiligen Fluiddurchlass 3, 4 abgewandten Stirnende mit dem Profilabschnitt 16 verbunden.

Anhand der Fig. 1 und 2 ist die Ventilvorrichtung 1 jeweils in einer Abdichtposition dargestellt, so dass die Einlassseite A gegenüber der Auslassseite B fluiddicht abgedichtet ist und umgekehrt. In dieser Abdichtposition liegen die Wandabschnitte 5, 6 jeweils mit ihrem Stirnende 10, 11 an den Seitenflächen 7 bzw. 8 an, so dass die Fluiddurchlässe 3, 4 jeweils fluiddicht abgedichtet sind. Die anhand der Fig. 1 und 2 ersichtliche Abdichtposition der Ventilvorrichtung 1 wird dann eingenommen, wenn zwischen der Einlassseite A und der Auslassseite B ein neutraler Fluiddruck herrscht. Dies ist beispielsweise bei einem Nichtgebrauch der Fluidleitungskomponente K der Fall, also dann, wenn das Lumen der Fluidleitungskomponente K weder in die eine noch in die andere Richtung durchströmt werden soll. Auf diese Weise wirkt die Ventilvorrichtung 1 insbesondere einem ungewollten Eindringen einer Körperflüssigkeit, wie beispielsweise Blut, ausgehend von der Auslassseite B in die Einlassseite A entgegen, so dass ein Verstopfen der Fluidleitungskomponente K vermieden werden kann.

Nachfolgend wird die Funktionsweise der Ventilvorrichtung 1 bei einem zwischen der Einlassseite A und der Auslassseite B vorherrschenden Fluidüberdruck sowie einem Fluidunterdruck näher erläutert.

Ein Fluidüberdruck zwischen der Einlassseite A und der Auslassseite B kann beispielsweise dann auftreten, wenn eine medizinische Flüssigkeit ausgehend von der Einlassseite A durch die Fluidleitungskomponente K injiziert werden soll. Dabei wird der erste Wandabschnitt fluidüberdruckbedingt derart relativ zu dem Stegabschnitt 9 verlagert, dass das Stirnende 10 von der Seitenfläche 7 angehoben wird. Hierbei wird der schlitzförmige erste Fluiddurchlass 3 in Normalenrichtung der Seitenfläche 7 geweitet, so dass die medizinische Flüssigkeit ausgehend von der Einlassseite A in Richtung der Auslassseite B injiziert werden kann. Gleichzeitig bewirkt der Fluidüberdruck eine elastische Verlagerung des zweiten Wandabschnitts 6 gegenüber der Seitenfläche 8 dahingehend, dass das Stirnende 11 verstärkt entgegen der Normalenrichtung der Seitenfläche 8 gegen den Stegabschnitt 9 gepresst wird. Der schlitzförmige zweite Fluiddurchlass 4 wird hierbei - in Bezug auf einen neutralen Fluiddruck - verstärkt abgedichtet.

Ein Fluidunterdruck zwischen der Einlassseite A und der Auslassseite B kann beispielsweise dann auftreten, wenn eine Körperflüssigkeit ausgehend von der Auslassseite B durch die Fluidleitungskomponente K aspiriert werden soll. Dabei wird der zweite Wandabschnitt 6 fluidunterdruckbedingt derart relativ zu dem Stegabschnitt 9 elastisch verlagert, dass das Stirnende 11 in Normalenrichtung der Seitenfläche 8 von dem Stegabschnitt 9 angehoben wird. Dies bewirkt, dass der zweite Fluiddurchlass 4 in Normalenrichtung der Seitenfläche 8 aufgeweitet wird. Auf diese Weise kann die Körperflüssigkeit ausgehend von der Auslassseite B durch die Fluidleitungskomponente K aspiriert werden. Die Körperflüssigkeit fließt hierbei ausgehend von der Auslassseite B durch die Einlassöffnung 15 des Lumens 13 und gelangt weiter durch den zweiten Fluiddurchlass 4 auf die Einlassseite A. Gleichzeitig wird der erste Wandabschnitt 5 fluidunterdruckbedingt derart relativ zu dem Stegabschnitt 9 elastisch verlagert, dass das Stirnende 10 entgegen der Normalenrichtung der Seitenfläche 7 gegen den Stegabschnitt 9 gepresst wird. Auf diese Weise wird der erste Fluiddurchlass - in Bezug auf einen neutralen Fluiddruck - verstärkt abgedichtet.

Aufgrund der erfindungsgemäßen Gestaltung der Ventilvorrichtung 1 ist der erste Fluiddurchlass 3 unbeeinflusst von einer fluiddruckbedingten Deformation des zweiten Wandabschnitts 6 und der zweite Fluiddurchlass 4 ist unbeeinflusst von einer fluiddruckbedingten Deformation des ersten Wandabschnitts 5. Demzufolge kann die Ventilvorrichtung 1 insbesondere mittels einer entsprechenden konstruktiven Ausgestaltung der Wandabschnitte 5, 6 in Bezug auf einen bei einer Injektion und einer Aspiration zu erzielenden Volumenstrom und/oder diesbezügliche Öffnungs- bzw. Schließdrücke angepasst werden.

Die Ausführungsformen erfindungsgemäßer Ventilvorrichtungen 1 nach den Fig. 1 und 2 sowie 1a nach Fig. 3 weisen hinsichtlich ihrer strukturellen sowie funktionellen Merkmale einen im Wesentlichen übereinstimmenden Aufbau auf. Zur Vermeidung von Wiederholungen wird daher in Bezug auf die Ventilvorrichtung 1a auf die Offenbarung zu der Ventilvorrichtung 1 nach den Fig. 1 und 2 verwiesen. Nachfolgend wird lediglich auf die wesentlichen Unterschiede der Ventilvorrichtung 1a nach Fig. 3 gegenüber der Ventilvorrichtung 1 nach den Fig. 1 und 2 eingegangen. Funktions- und/oder baugleiche Teile und Abschnitte der Ventilvorrichtung 1a sind mit gleichen Bezugszeichen unter Hinzufügung des Kleinbuchstabens a versehen.

Die Ventilvorrichtung 1a unterscheidet sich im Wesentlichen dahingehend von der Ventilvorrichtung 1, dass die Wandabschnitte 5a, 6a derart unterschiedlich elastisch verlagerbar gestaltet sind, dass die Fluiddurchlässe 3a, 4a bei betragsmäßig unterschiedlichen Fluiddrücken freigegeben und/oder abgedichtet sind. Zu diesem Zweck weisen die Wandabschnitte 5a, 6a unterschiedliche Wandstärken bzw. Wandstärkenverläufe auf. So ist der erste Wandabschnitt 5a, wie anhand Fig. 3 ersichtlich ist, im Vergleich zu dem Wandabschnitt 6a dünnwandiger gestaltet. Demzufolge ist der erste Wandabschnitt 5a im Vergleich zu dem zweiten Wandabschnitt 6a elastisch nachgiebiger gestaltet. Alternativ oder zusätzlich ist es auch möglich, dass die Wandabschnitte 5a, 6a aus unterschiedlichen Werkstoffen gefertigt sind. Beispielsweise kann der zweite Wandabschnitt 6a aus einem - im Vergleich zu einem Werkstoff des ersten Wandabschnitts 5a - steiferen Werkstoff mit einem insoweit höheren E-Modul gefertigt sein oder umgekehrt. Wie weiter anhand Fig. 3 ersichtlich ist, sind die Wandabschnitte 5a, 6a betragsmäßig unterschiedlich gegenüber dem Stegabschnitt 9a geneigt. Der erste Wandabschnitt 5a ist in einem Winkel von in etwa 20°, der zweite Wandabschnitt 6a in einem Winkel von in etwa 25° gegenüber dem Stegabschnitt 9a geneigt. Vorliegend sind die Stegabschnitte 5a, 6a in etwa gleich lang erstreckt. Es ist jedoch auch möglich, dass die Wandabschnitte 5a, 6a unterschiedlich längserstreckt sind. Infolge der betragsmäßig unterschiedlichen Neigung und/oder Längserstreckung ist eine - senkrecht zur Einlassseite A - projizierte Druckfläche M des ersten Wandabschnitts 5a unterschiedlich zu einer - senkrecht zu der Auslassseite B - projizierten Druckfläche N des zweiten Wandabschnitts 6a. Dies bewirkt alternativ oder zusätzlich zu einer unterschiedlich elastischen Ausgestaltung im Hinblick auf die Wandstärken und/oder Werkstoffe, dass die Fluiddurchlässe 3a, 4a bei betragsmäßig unterschiedlichen Fluiddrücken freigegeben und/oder abgedichtet sind.

Anhand Fig. 4 ist eine weitere mögliche Einbausituation der Ventilvorrichtungen 1, 1a in einer Fluidleitungskomponente L in Form eines - stark vereinfacht dargestellten - Dreiwegehahns dargestellt.

## Patentansprüche

1. Ventilvorrichtung (1, 1a) für ein medizinisches Fluidleitungssystem mit
- einem Grundkörper (2, 2a), der wenigstens abschnittsweise weichelastisch gestaltet ist, und
- einem ersten Fluiddurchlass (3, 3a) sowie einem zweiten Fluiddurchlass (4, 4a), die jeweils zwischen einer Einlassseite (A) und einer Auslassseite (B) des Grundkörpers (2, 2a) erstreckt sind,
- wobei der Grundkörper (2, 2a) derart gestaltet und die Fluiddurchlässe (3, 3a, 4, 4a) derart angeordnet sind, dass
- der erste Fluiddurchlass (3, 3a) bei einem Fluidüberdruck zwischen der Einlassseite (A) und der Auslassseite (B) mittels einer fluidüberdruckbedingten elastischen Deformation des Grundkörpers (2, 2a) freigegeben ist und der zweite Fluiddurchlass (4, 4a) fluiddicht abgedichtet ist, und
- der zweite Fluiddurchlass (4, 4a) bei einem Fluidunterdruck zwischen der Einlassseite (A) und der Auslassseite (B) mittels einer fluidunterdruckbedingten elastischen Deformation des Grundkörpers (2, 2a) freigegeben ist und der erste Fluiddurchlass (3, 3a) fluiddicht abgedichtet ist, und
- die Fluiddurchlässe (3, 3a, 4, 4a) bei einem neutralen Fluiddruck zwischen der Einlassseite (A) und der Auslassseite (B) fluiddicht abgedichtet sind,
wobei der Grundkörper (2, 2a) einen ersten Wandabschnitt (5, 5a) und einen zweiten Wandabschnitt (6, 6a) aufweist, die jeweils relativ zu einem im Wesentlichen formstabilen Stegabschnitt (9, 9a) des Grundkörpers (2, 2a) fluiddruckbedingt elastisch verlagerbar sind, **dadurch gekennzeichnet, dass** die Wandabschnitte (5, 5a, 6, 6a) unter Ausbildung jeweils eines der Fluiddurchlässe (3, 3a, 4, 4a) an einander gegenüberliegenden Seitenflächen (7, 7a, 8, 8a) des Stegabschnitts (9, 9a) und somit getrennt voneinander angeordnet sind, so dass der erste Fluiddurchlass (3, 3a) im Wesentlichen unbeeinflusst von einer fluiddruckbedingten Deformation des zweiten Wandabschnitts (6, 6a) ist und der zweite Fluiddurchlass (4, 4a) im Wesentlichen unbeeinflusst von einer fluiddruckbedingten Deformation des ersten Wandabschnitts (5, 5a) ist.

2. Ventilvorrichtung (1, 1a) nach Anspruch 1, **dadurch gekennzeichnet, dass** die Wandabschnitte (5, 5a, 6, 6a) - in Bezug auf einen abgedichteten Zustand - jeweils wenigstens abschnittsweise nach Art einer Dichtlippe an dem Stegabschnitt (9, 9a) anliegen, wobei jeweils einer der Fluiddurchlässe (5, 5a, 6, 6a) in Form eines Schlitzes zwischen dem Stegabschnitt (9, 9a), insbesondere der jeweiligen Seitenflächen (7, 7a, 8, 8a), und dem jeweiligen Wandabschnitt (5, 5a, 6, 6a) ausgebildet ist.

3. Ventilvorrichtung (1, 1a) nach Anspruch 1 oder 2, **dadurch gekennzeichnet, dass** der erste Fluiddurchlass (3, 3a) zwischen einem der Auslassseite (B) zugewandten Stirnende (10, 10a) des ersten Wandabschnitts (5, 5a) und dem Stegabschnitt (9, 9a) ausgebildet ist und der zweite Fluiddurchlass (4, 4a) zwischen einem der Einlassseite (A) zugewandten Stirnende (11, 11a) des zweiten Wandabschnitts (6, 6a) und dem Stegabschnitt (9, 9a) ausgebildet ist.

4. Ventilvorrichtung (1, 1a) nach einem der vorhergehenden Ansprüche, **dadurch gekennzeichnet, dass** die Wandabschnitte (5, 5a, 6, 6a) den Stegabschnitt (9, 9a), insbesondere die jeweilige Seitenfläche (7, 7a, 8, 8a), wenigstens abschnittsweise gewölbt unter Ausbildung jeweils eines Lumens (12, 12a, 13, 13a) überspannen, wobei die Lumen (12, 12a, 13, 13a) jeweils einends in den jeweiligen Fluiddurchlass (3, 3a, 4, 4a) münden.

5. Ventilvorrichtung (1, 1a) nach Anspruch 4, **dadurch gekennzeichnet, dass** die Lumen (12, 12a, 13, 13a) jeweils nach Art einer Strömungsdüse geformt und derart strömungstechnisch entgegengesetzt zueinander orientiert sind, dass eine Einlassöffnung (14, 14a) des dem ersten Wandabschnitt (5, 5a) zugeordneten Lumens (12, 12a) in Richtung der Einlassseite (A) orientiert ist und eine Einlassöffnung (15, 15a) des dem zweiten Wandabschnitt (6, 6a) zugeordneten Lumens (13, 13a) in Richtung der Auslassseite (B) orientiert ist, wobei die Fluiddurchlässe (3, 3a, 4, 4a) jeweils eine Auslassöffnung des jeweiligen Lumens bilden.

6. Ventilvorrichtung (1, 1a) nach einem der vorhergehenden Ansprüche, **dadurch gekennzeichnet, dass** der Stegabschnitt (9, 9a) - im Vergleich zu den Wandabschnitten (5, 5a, 6, 6a) - steif gestaltet ist und im Wesentlichen entlang einer Mittellängsachse (C) des Grundkörpers (2, 2a) in Form einer ebenen Platte erstreckt ist.

7. Ventilvorrichtung (1a) nach einem der vorhergehenden Ansprüche, **dadurch gekennzeichnet, dass** die Wandabschnitte (5a, 6a) derart unterschiedlich elastisch verlagerbar gestaltet sind, dass die Fluiddurchlässe (3a, 4a) bei betragsmäßig unterschiedlichen Fluiddrücken freigegeben und/oder abgedichtet sind.

8. Ventilvorrichtung (1a) nach Anspruch 7, **dadurch gekennzeichnet, dass** die Wandabschnitte (5a, 6a) unterschiedliche Wandstärken, Wandstärkenverläufe und/oder elastische Eigenschaften aufweisen.

9. Ventilvorrichtung (1a) nach Anspruch 7 oder 8, **dadurch gekennzeichnet, dass** die Wandabschnitte (5a, 6a) betragsmäßig unterschiedlich gegenüber dem Stegabschnitt (9a) geneigt und/oder unterschiedlich längserstreckt sind, so dass eine - senkrecht zu der Einlassseite (A) - projizierte Druckfläche (M) des ersten Wandabschnitts (5a) unterschiedlich zu einer - senkrecht zu der Auslassseite (B) - projizierten Druckfläche (N) des zweiten Wandabschnitts (6a) ist.

10. Ventilvorrichtung (1, 1a) nach einem der vorhergehenden Ansprüche, **dadurch gekennzeichnet, dass** der Grundkörper (2, 2a) einen ringförmigen Profilabschnitt (16, 16a) aufweist, der zur formschlüssigen Verbindung mit einem Innendurchmesser (D) einer medizinischen Fluidleitungskomponente (K) vorgesehen ist.

11. Ventilvorrichtung (1, 1a) nach Anspruch 10, **dadurch gekennzeichnet, dass** der Profilabschnitt (16, 16a) den Stegabschnitt (9, 9a) - in Bezug auf eine Längserstreckung des Stegabschnitts (9, 9a) - in etwa mittig wenigstens abschnittsweise in Umfangsrichtung umgreift.

12. Ventilvorrichtung (1, 1a) nach Anspruch 10 oder 11, **dadurch gekennzeichnet, dass** die Wandabschnitte (5, 5a, 6, 6a) jeweils an einem dem jeweiligen Fluiddurchlass (3, 3a, 4, 4a) abgewandten Stirnende mit dem Profilabschnitt (16) verbunden sind.

13. Ventilvorrichtung (1, 1a) nach einem der vorhergehenden Ansprüche, **dadurch gekennzeichnet, dass** der Grundkörper (2, 2a) einstückig ausgebildet ist.

## Claims

1. Valve device (1, 1a) for a medical fluid line system, with:
- a main body (2, 2a), which is configured to be soft-elastic at least in part, and
- a first fluid passage (3, 3a) and a second fluid passage (4, 4a) which each extend between an inlet side (A) and an outlet side (B) of the main body (2, 2a),
- wherein the main body (2, 2a) is configured in such a way and the fluid passages (3, 3a, 4, 4a) are arranged in such a way that,
- in the event of a fluid overpressure between the inlet side (A) and the outlet side (B), the first fluid passage (3, 3a) is freed by means of a fluid-overpressure-induced elastic deformation of the main body (2, 2a) and the second fluid passage (4, 4a) is sealed off in a fluid-tight manner, and,
- in the event of a fluid underpressure between the inlet side (A) and the outlet side (B), the second fluid passage (4, 4a) is freed by means of a fluid-underpressure-induced elastic deformation of the main body (2, 2a) and the first fluid passage (3, 3a) is sealed off in a fluid-tight manner, and,
- in the event of a neutral fluid pressure between the inlet side (A) and the outlet side (B), the fluid passages (3, 3a, 4, 4a) are sealed off in a fluid-tight manner,
wherein the main body (2, 2a) has a first wall portion (5, 5a) and a second wall portion (6, 6a) which are each elastically displaceable, under the effect of a fluid pressure, relative to a substantially dimensionally stable web portion (9, 9a) of the main body (2, 2a),
**characterized in that**
the wall portions (5, 5a, 6, 6a) are arranged on mutually opposite side faces (7, 7a, 8, 8a) of the web portion (9, 9a), thereby each forming one of the fluid passages (3, 3a, 4, 4a), and thus separately from one another, such that the first fluid passage (3, 3a) is substantially uninfluenced by a fluid-pressure-induced deformation of the second wall portion (6, 6a), and the second fluid passage (4, 4a) is substantially uninfluenced by a fluid-pressure-induced deformation of the first wall portion (5, 5a).

2. Valve device (1, 1a) according to claim 1, **characterized in that** the wall portions (5, 5a, 6, 6a), with respect to a sealed-off state, each bear at least in part on the web portion (9, 9a) in the manner of a sealing lip, wherein in each case one of the fluid passages (5, 5a, 6, 6a) is configured in the form of a slit between the web portion (9, 9a), in particular of the respective side faces (7, 7a, 8, 8a), and the respective wall portion (5, 5a, 6, 6a).

3. Valve device (1, 1a) according to claim 1 or 2, **characterized in that** the first fluid passage (3, 3a) is formed between a front end (10, 10a) of the first wall portion (5, 5a), directed towards the outlet side (B), and the web portion (9, 9a), and the second fluid passage (4, 4a) is formed between a front end (11, 11a) of the second wall portion (6, 6a), directed towards the inlet side (A), and the web portion (9, 9a).

4. Valve device (1, 1a) according to any of the preceding claims, **characterized in that** the wall portions (5, 5a, 6, 6a) span the web portion (9, 9a), in particular the respective side face (7, 7a, 8, 8a), at least partially in a curve in order to form in each case a lumen (12, 12a, 13, 13a), wherein the lumina (12, 12a, 13, 13a) each open out at one end into the respective fluid passage (3, 3a, 4, 4a).

5. Valve device (1, 1a) according to claim 4, **characterized in that** the lumina (12, 12a, 13, 13a) are each shaped in the manner of a flow nozzle and are oriented in opposite directions relative to each other fluidically, in such a way that an inlet opening (14, 14a) of the lumen (12, 12a) assigned to the first wall portion (5, 5a) is oriented in the direction of the inlet side (A), and an inlet opening (15, 15a) of the lumen (13, 13a) assigned to the second wall portion (6, 6a) is oriented in the direction of the outlet side (B), wherein the fluid passages (3, 3a, 4, 4a) each form an outlet opening of the respective lumen.

6. Valve device (1, 1a) according to any of the preceding claims, **characterized in that** the web portion (9, 9a) is stiff, compared to the wall portions (5, 5a, 6, 6a), and extends in the form of a flat plate substantially along a central longitudinal axis (C) of the main body (2, 2a).

7. Valve device (1a) according to any of the preceding claims, **characterized in that** the wall portions (5a, 6a) are configured to be elastically displaceable to different extents, in such a way that the fluid passages (3a, 4a) are freed and/or sealed off at quantitatively different fluid pressures.

8. Valve device (1a) according to claim 7, **characterized in that** the wall portions (5a, 6a) have different wall thicknesses, wall thickness profiles and/or elastic properties.

9. Valve device (1a) according to claim 7 or 8, **characterized in that** the wall portions (5a, 6a) are inclined to different extents and/or extend by different lengths relative to the web portion (9a), such that a pressure surface (M) of the first wall portion (5a), projected perpendicularly with respect to the inlet side (A), is different than a pressure surface (N) of the second wall portion (6a), projected perpendicularly with respect to the outlet side (B).

10. Valve device (1, 1a) according to any of the preceding claims, **characterized in that** the main body (2, 2a) has an annular profile portion (16, 16a) which is provided for form-fit connection to an internal diameter (D) of a medical fluid line component (K).

11. Valve device (1, 1a) according to claim 10, **characterized in that** the profile portion (16, 16a) circumferentially engages at least in part around the web portion (9, 9a), approximately centrally with respect to a longitudinal extent of the web portion (9, 9a).

12. Valve device (1, 1a) according to claim 10 or 11, **characterized in that** the wall portions (5, 5a, 6, 6a) are each connected to the profile portion (16) at a front end directed away from the respective fluid passage (3, 3a, 4, 4a).

13. Valve device (1, 1a) according to any of the preceding claims, **characterized in that** the main body (2, 2a) is configured in one piece.

## Revendications

1. Dispositif de vanne (1, 1a) pour un système médical de conduite de fluide doté
- d'un corps de base (2, 2a), lequel est au moins partiellement conçu comme souple et élastique, et
- d'un premier passage de fluide (3, 3a) ainsi qu'un deuxième passage de fluide (4, 4a), lesquels s'étendent entre un côté d'entrée (A) et un côté de sortie (B) du corps de base (2, 2a),
- dans lequel le corps de base (2, 2a) est conçu et les passages de fluide (3, 3a, 4, 4a) agencés de telle sorte que
• le premier passage de fluide (3, 3a) est dégagé lors d'une surpression de fluide entre le côté d'entrée (A) et le côté de sortie (B) au moyen d'une déformation élastique du corps de base (2, 2a) conditionnée par une surpression de fluide et le deuxième passage de fluide (4, 4a) est obturé de manière étanche au fluide, et
• le deuxième passage de fluide (4, 4a) est dégagé lors d'une dépression de fluide entre le côté d'entrée (A) et le côté de sortie (B) au moyen d'une déformation élastique du corps de base (2, 2a) conditionnée par une dépression de fluide et le premier passage de fluide (3, 3a) est obturé de manière étanche au fluide, et
• les passages de fluide (3, 3a, 4, 4a) sont obturés de manière étanche au fluide en cas de pression de fluide neutre entre le côté d'entrée (A) et le côté de sortie (B),
dans lequel le corps de base (2, 2a) comporte une première section de paroi (5, 5a) et une deuxième section de paroi (6, 6a), lesquelles sont élastiquement déplaçables respectivement par rapport à une section nervurée (9, 9a) essentiellement indéformable du corps de base (2, 2a) en fonction de la pression de fluide, **caractérisé en ce que** les sections de paroi (5, 5a, 6, 6a) sont, afin de réaliser respectivement un des passages de fluide (3, 3a, 4, 4a), agencées sur des surfaces latérales (7, 7a, 8, 8a) de la section nervurée (9, 9a) opposées l'une à l'autre et ainsi séparées l'une de l'autre, de sorte que le premier passage de fluide (3, 3a) n'est essentiellement pas affecté par une déformation de la deuxième section de paroi (6, 6a) conditionnée par la pression de fluide et le deuxième passage de fluide (4, 4a) n'est essentiellement pas affecté par une déformation de la première section de paroi (5, 5a) conditionnée par la pression de fluide.

2. Dispositif de vanne (1, 1a) selon la revendication 1, **caractérisé en ce que** les sections de paroi (5, 5a, 6, 6a), par rapport à un état obturé, s'appuient sur la section nervurée (9, 9a) respectivement au moins en partie selon le type d'une lèvre d'étanchéité, dans lequel un des passages de fluide (5, 5a, 6, 6a) est respectivement réalisé sous la forme d'une fente entre la section nervurée (9, 9a), en particulier des surfaces latérales respectives (7, 7a, 8, 8a), et la section de paroi respective (5, 5a, 6, 6a).

3. Dispositif de vanne (1, 1a) selon la revendication 1 ou 2, **caractérisé en ce que** le premier passage de fluide (3, 3a) est réalisé entre une extrémité frontale (10, 10a) de la première section de paroi (5, 5a) faisant face au côté de sortie (B) et la section nervurée (9, 9a) et le deuxième passage de fluide (4, 4a) est réalisé entre une extrémité frontale (11, 11a) de la deuxième section de paroi (6, 6a) faisant face au côté d'entrée (A) et la section nervurée (9, 9a).

4. Dispositif de vanne (1, 1a) selon l'une des revendications précédentes, **caractérisé en ce que** les sections de paroi (5, 5a, 6, 6a) enjambent de manière au moins partiellement courbées la section nervurée (9, 9a), en particulier les surfaces latérales respectives (7, 7a, 8, 8a), pour former respectivement une lumière (12, 12a, 13, 13a), dans lequel la lumière (12, 12a, 13, 13a) débouche respectivement sur une extrémité dans le passage de fluide respectif (3, 3a, 4, 4a).

5. Dispositif de vanne (1, 1a) selon la revendication 4, **caractérisé en ce que** les lumières (12, 12a, 13, 13a) sont respectivement selon le type d'une buse d'écoulement et sont fluidiquement orientées en opposition les unes par rapport aux autres de telle sorte qu'une ouverture d'entrée (14, 14a) de la lumière (12, 12a) ordonnée à la première section de paroi (5, 5a) est orientée dans la direction du côté d'entrée (A) et une ouverture d'entrée (15, 15a) de la lumière (13, 13a) ordonnée à la deuxième section de paroi (6, 6a) est orientée dans la direction du côté de sortie (B), dans lequel les passages de fluide (3, 3a, 4, 4a) forment respectivement une ouverture de sortie de la lumière respective.

6. Dispositif de vanne (1, 1a) selon l'une des revendications précédentes, **caractérisé en ce que** la section nervurée (9, 9a) est conçue de manière rigide comparée aux sections de paroi (5, 5a, 6, 6a) et s'étend essentiellement le long d'un axe longitudinal médian (C) du corps de base (2, 2a) sous forme d'un panneau plat.

7. Dispositif de vanne (1a) selon l'une des revendications précédentes, **caractérisé en ce que** les sections de paroi (5a, 6a) sont conçues pour être élastiquement déplaçables différemment de telle sorte que les passages de fluide (3a, 4a) sont dégagés et/ou obturés à des pressions de fluide quantitativement différentes.

8. Dispositif de vanne (1a) selon la revendication 7, **caractérisé en ce que** les sections de paroi (5a, 6a) possèdent différentes épaisseurs de paroi, différents profils d'épaisseur de paroi et/ou différentes propriétés élastiques.

9. Dispositif de vanne (1a) selon la revendication 7 ou 8, **caractérisé en ce que** les sections de paroi (5a, 6a) sont inclinées de manière quantitativement différente par rapport à la section nervurée (9a) et/ou différemment allongées, de sorte qu'une surface de pression (M) de la première section de paroi (5a) projetée perpendiculairement au côté d'entrée (A) est différente d'une surface de pression (N) de la deuxième section de paroi (6a) projetée perpendiculairement au côté de sortie (B).

10. Dispositif de vanne (1, 1a) selon l'une des revendications précédentes, **caractérisé en ce que** le corps de base (2, 2a) possède une section de profil annulaire (16, 16a), laquelle est prévue pour le raccordement par complémentarité de formes avec un diamètre interne (D) d'un composant de conduite de fluide médical (K).

11. Dispositif de vanne (1, 1a) selon la revendication 10, **caractérisé en ce que** la section de profil (16, 16a) entoure au moins partiellement dans la direction circonférentielle la section nervurée (9, 9a) à peu près au milieu, par rapport à une étendue longitudinale de la section nervurée (9, 9a).

12. Dispositif de vanne (1, 1a) selon la revendication 10 ou 11, **caractérisé en ce que** les sections de paroi (5, 5a, 6, 6a) sont reliées respectivement à la section de profil (16) à une extrémité frontale se détournant du passage de fluide respectif (3, 3a, 4, 4a).

13. Dispositif de vanne (1, 1a) selon l'une des revendications précédentes, **caractérisé en ce que** le corps de base (2, 2a) est réalisé d'une pièce.
